# EUROPEAN PATENT APPLICATION

(11) **EP 3 622 903 A1**
(43) Date of publication of application: **18.03.2020**
(21) Application number: 18798990.0
(22) Date of filing: 08.05.2018
(51) Int. Cl.: A61B 17/28, B25J 17/00

(54) **MANIPULATOR**

(30) Priority: 08.05.2017 JP 2017092574
(71) Applicant: Kyushu University, National University Corporation, Nishi-ku Fukuoka-shi Fukuoka 819-0395 (JP)
(72) Inventor: ARATA, Jumpei, Fukuoka-shi Fukuoka 819-0395 (JP); HASHIZUME, Makoto, Fukuoka-shi Fukuoka 819-0395 (JP)
(74) Representative: Brevalex
(86) International application number: PCT/JP2018/017843
(87) International publication number: WO 2018/207799

(57) **Abstract**

A manipulator that deforms a plate spring by pushing and pulling the plate spring and operates an operation portion with multiple degrees of freedom includes a pair of flexible operating bodies (first flexible operating body and second flexible operating body) including a horizontal plate spring, a curved vertical plate spring, a shaft body, and the operation portion. The pair of flexible operating bodies overlaps such that a second flexible operating body on one side is opposite to a first flexible operating body on the other side, and is linked to each other to be rotatable by a pin penetrating respective shaft bodies. In a holder that supports both ends of the pin, a pair of outer horizontal plate springs (first outer horizontal plate spring and second outer horizontal plate spring) that has the horizontal plate springs of each of the pair of flexible operating bodies to be interposed in parallel between the holder and extends in the same direction as the horizontal plate spring is integrally formed.

## Description

### TECHNICAL FIELD

The present disclosure relates to a manipulator.

### BACKGROUND ART

In recent years, minimally invasive surgery represented, for example, by laparoscopic surgery attracts attention as one method of a surgical operation. In the minimally invasive surgery, surgical instruments such as an endoscope or a forceps are inserted into a body through a small incision hole of approximately 5 mm to 10 mm opened in a body surface, and surgery is performed inside the body. Therefore, compared to ordinary surgical operations (for example, open chest surgery or open abdominal surgery), there is an advantage that damage to the body of a patient at the time of surgery and pain after surgery can be minimized.

Since the forceps used for minimally invasive surgery is restricted in postures thereof by incision holes in the body of the patient, the forceps can only approach from a limited direction to a target lesion. In order to easily perform work, such as griping and ligating, under such conditions, there is a demand for the development of bending forceps (that is, multi-degree-of-freedom manipulator) configured to bend the tip end part of the forceps in a plurality of directions.

As a product of this type of multi-degree-of-freedom manipulator, for example, "da Vinci surgical system" by Intuitive Surgical, Inc. is known (see Non-Patent Literature 1). In the multi-degree-of-freedom manipulator of Non-Patent Literature 1, a wire is employed as a tool for transmitting power from a driving device. By winding up the wire with the driving device, bending of a joint, opening and closing of a gripping portion, and the like are realized.

Patent Literature 1 is also cited as a related art regarding a multi-degree-of-freedom manipulator, for example. In the multi-degree-of-freedom manipulator disclosed in Patent Literature 1, a link mechanism is employed as a tool for transmitting power from a driving device.

### CITATION LIST

### PATENT LITERATURE

Patent Literature 1: Japanese Patent No. 4460890

### NON-PATENT LITERATURE

Non-Patent Literature 1: Gary S. Guthart and J. Kenneth Salisbury, Jr., "The Intuitive (TM) Telesurgery System: Overview and Application", Proceedings of the 2000 IEEE International Conference on Robotics & Automation San Francisco, CA April 2000, pp. 618-621, 2000

### SUMMARY OF INVENTION

### TECHNICAL PROBLEM

However, the wire driving that serves as the power transmission method employed in Non-Patent Literature 1 has the following problems.

Firstly, since there is a concern that the wire may be "stretched" or "broken", the wire must be replaced frequently. For example, in the above-described "da Vinci surgical system", it is necessary to replace wires for approximately 10 surgeries. In addition, since the wire is wound around a plurality of gears or pulleys, it takes a lot of time and effort in detaching and mounting. Accordingly, an increase in running costs and maintenance load are caused.

Secondly, since the wire stretches and contracts, there is a limit to the control accuracy of the joint or the gripping portion. In addition, there is also a disadvantage that the wire can transmit power only in one direction (drawing direction).

Thirdly, there is a problem that it is difficult to sterilize and clean the wire. Therefore, in the multi-degree-of-freedom manipulator of the related art, preoperative and postoperative sterilization and cleaning work is extremely complicated.

In addition, the link mechanism that serves as the power transmission method employed in Patent Literature 1 has the following problems.

Firstly, in a case where a plurality of link mechanisms configured of a plurality of links are provided, the number of components increases, it is difficult to reduce the size and weight, and the product costs increase.

Secondly, when a rotation operation is performed by the plurality of link mechanisms, a bending radius (in other words, swing radius) of a capturing device (for example, first supporting body 16 of Patent Literature 1) becomes large, and it is difficult to smoothly move so as to approach the target lesion at the time of surgery performed on the narrow site. This problem also occurs in wire driving that uses a gear or a pulley.

In addition, when the number of members constituting the gripping portion increases, the number of joint portions increases. Increasing the number of joint portions becomes an obstacle to reducing the diameter of the gripping portion when applied to, for example, neurosurgery. When the joint portion has a predetermined outer diameter (for example, approximately 4 mm or less) that allows the manipulator to be applied to neurosurgery, it is difficult to secure the joint strength. In particular, when titanium or a titanium alloy that does not have good bonding properties with different metals is used as a material, there is a possibility that the joint portion is detached. Therefore, there is a demand for realizing a structure that can be reduced in diameter and that does not easily cause separation of components even in a small diameter applicable to neurosurgery. Technical measures for this request are not considered in the related art such as Non-Patent Literature 1 or Patent Literature 1 described above.

The present disclosure is devised in view of the above-described circumstances in the related art, and an object thereof is to provide a manipulator that can eliminate a wire as a tool for transmitting power to a tip end portion that performs treatment on a target lesion, can be reduced in size, weight, and cost with a small number of components, and can be easily approached to the target lesion.

### SOLUTION TO PROBLEM

The present disclosure provides a manipulator including a pair of flexible operating bodies including a rectangular horizontal plate spring, a curved vertical plate spring which stands upright with a plate surface perpendicular to the plate surface of the horizontal plate spring, in which a proximal end of the curved vertical plate spring is connected to one end of the horizontal plate spring in a longitudinal direction, the curved vertical plate spring protrudes to one side in a plate width direction of the horizontal plate spring, and extends along the longitudinal direction of the horizontal plate spring, and in which a tip end of the curved vertical plate spring in an extending direction is a bent portion bent in a direction opposite to a direction of the protruding, a shaft body which is connected to a tip end of the bent portion and is supported to be rotatable at a rotation center perpendicular to the plate surface of the horizontal plate spring, and an operation portion which is provided to protrude in a radial direction from an outer periphery of the shaft body, and a holder which supports both ends of a pin passing through the shaft body of each of the pair of flexible operating bodies and links the pair of flexible operating bodies to be rotatable about the rotation center, in which the holder has the horizontal plate springs of each of the pair of flexible operating bodies to be interposed in parallel between the holder, and a pair of outer horizontal plate springs extending in the same direction as the horizontal plate spring is integrally formed with the holder.

According to this manipulator, the horizontal plate spring, the curved vertical plate spring, and the shaft body are disposed in series on the same plane to configure a flexible operating body. The curved vertical plate spring is connected to the horizontal plate spring in a direction in which the plate surface stands upright. When the other end of the horizontal plate spring in the longitudinal direction is pressed in the direction along the longitudinal direction, in a case where the shaft body is supported so as to be immovable within the same plane as described above, the flexible operating body is deformed in a direction in which the curved vertical plate spring is further curved. Since the horizontal plate spring and the plate surface of the curved vertical plate spring are orthogonal to each other, the deformation direction of the curved vertical plate spring changes by 90 degrees. In the curved vertical plate spring, most of the internal stress accumulated by the deformation is an elastic restoring force. Apart of the elastic restoring force acts as a component force in a tangent direction of the outer periphery of the shaft body which generates a moment with respect to the shaft body.

Since the protruding directions of the curved vertical plate springs of the pair of flexible operating bodies are opposite to each other, by pushing and pulling the other ends of the horizontal plate springs in the longitudinal direction (that is, the side opposite to the operation portion side) at the same time, each operation portion can be moved close to and away from each other. That is, when the operation portion is a capturing device such as a forceps or a pincer in laparoscopic surgery, for example, pinching becomes possible. In addition, in the manipulator, by pushing or pulling the other end of the pair of horizontal plate springs in the longitudinal direction (that is, the side opposite to the operation portion side) at the same time in the opposite directions, the pair of operation portions can be rotated about the rotation center of the shaft body in the same direction. That is, when the operation portion is a capturing device such as a forceps or a pincer in laparoscopic surgery, for example, it is possible to rotate in forward and reverse directions while the capturing device is closed.

In the manipulator, a pair of outer horizontal plate springs is disposed on the outside of the pair of horizontal plate springs so as to interpose the pair of horizontal plate springs between the outer horizontal plate springs. That is, a pair of horizontal plate springs and the pair of outer horizontal plate springs are arranged in four layers. The pair of horizontal plate springs and the pair of outer horizontal plate springs are pushed and pulled independently of each other. One end of the pair of outer horizontal plate springs in the longitudinal direction (that is, the operation portion side) extends from the holder. In the manipulator, when the pair of outer horizontal plate springs is pushed and pulled in the opposite direction from each other, the pair of outer horizontal plate springs is deformed (bent) in a direction of being inclined to one outer horizontal plate spring side together, or the pair of outer horizontal plate springs is deformed (bent) in a direction of being inclined to the other outer horizontal plate spring side together. The holder is displaced (tilted) by the bending of the pair of outer horizontal plate springs. At this time, the pair of horizontal plate springs disposed between the pair of outer horizontal plate springs also bends passively in the same direction.

In the manipulator, the pair of outer horizontal plate springs is formed integrally with the holder. That is, in the holder, the holder body and the outer horizontal plate spring are integrally formed without having a joint portion. The holder does not have a joint portion by a fixture, a caulking portion, an adhesive, or the like. In the related art, when the holder body is made of stainless steel, and the outer horizontal plate spring is made of titanium or a titanium alloy, due to the difficulty of welding, the holder body and the outer horizontal plate spring are connected and integrated by the pin or the like (separate members are combined into one). When the holder body is integrated by fixing each of the pair of outer horizontal plate springs with a fixture such as the pin, for example, the holder needs at least five components (one holder body, two outer horizontal plate springs, and two fixtures). In contrast, the integrally formed holder needs only one component. In the integrally formed holder, wear, backlash, or accuracy degradation caused by a joining structure using a fixture or the like does not occur. In addition, body fluids such as blood do not enter the joined structure and can be easily cleaned, and can be used safely in the next and subsequent operations by being sufficiently dried. Furthermore, it is not necessary to downsize individual components when a plurality of separate components are integrated. Therefore, compare to the manipulator having the same size, since the integrally formed holder eliminates a plurality of small components, the risk of strength reduction and the risk of disconnection can be avoided. Long-term use is also possible. As a result, the manipulator in which the holder is integrally formed has a great advantage particularly in the case of a small diameter (outer diameter of approximately 4 mm or less) applied to neurosurgery or like that requires miniaturization.

In addition, the present disclosure provides a manipulator in which the pair of outer horizontal plate springs extends from a cylindrical outer peripheral surface of the holder in a direction along a generatrix and has an arc-shaped outer peripheral surface having the same radius of curvature as the cylindrical outer peripheral surface.

According to this manipulator, the holder body has the cylindrical outer peripheral surface, and the pair of outer horizontal plate springs is extended from the cylindrical outer peripheral surface in a direction along the generatrix. The cylindrical outer peripheral surface and the arc-shaped outer peripheral surface of each outer horizontal plate spring have the same radius of curvature. That is, the holder body and the pair of outer horizontal plate springs can be processed from the same metal tube material without providing a joint portion. The holder uses a material (metal tube material) having a circular inner and outer diameter, removes a part thereof and leaves the other part, and accordingly, different rigidity (spring property) can be expressed at a desired site. In the manipulator to be applied to neurosurgery, the outer diameter of the holder inserted inside the outer tube (outer diameter of approximately 4 mm or less) is approximately 3 mm or less. The holder made of titanium or titanium alloy having such a small diameter can be manufactured by cutting a metal tube material by wire cut electric discharge machining, for example.

In addition, the present disclosure provides a manipulator in which a flexible portion that is thinner and narrower than both ends of the outer horizontal plate spring in the longitudinal direction is formed at a central portion of the outer horizontal plate spring in an extending direction.

According to this manipulator, each outer horizontal plate spring is formed with a site that is thinner and narrower than both ends in the longitudinal direction (that is, the flexible portion) is formed at the central portion in the extending direction. The flexible portion has a smaller cross-sectional area than both ends in the longitudinal direction. Therefore, in the outer horizontal plate spring, the stress acting on both ends in the longitudinal direction concentrates on the flexible portion at the central portion in the extending direction and is bent (elastically deformed). In this manner, by controlling the thickness and the plate width of the outer horizontal plate spring formed integrally with the holder body, the holder can be a single component made of the same material, and can express spring properly only at a desired position.

In addition, the present disclosure provides a manipulator in which the pair of outer horizontal plate springs is disposed inside an outer tube such that a part in an extending direction is in contact with the inner wall surface of the outer tube.

According to this manipulator, the pair of outer horizontal plate springs is deformed (bent) in a direction inclined toward one outer horizontal plate spring side or in a direction inclined toward the other outer horizontal plate spring. Since the pair of outer horizontal plate springs is in contact with the inner wall surface of the outer tube, bending is restricted inside the outer tube. The pair of outer horizontal plate springs is bent at a part where most of the pair of outer horizontal plate springs protrudes from the outer tube. Therefore, in the pair of outer horizontal plate springs, the open end of the outer tube can be regarded as a bending start end. That is, the opening portion of the outer tube serves as a fulcrum for supporting the outer horizontal plate spring on the bending side. In this manner, in the manipulator, a part of the outer horizontal plate spring is made into the inner wall surface of the outer tube, and the open end of the outer tube is used as a fulcrum. Accordingly, the outer horizontal plate spring can suppress the useless expansion of a bending radius. The fulcrum moves on the outer horizontal plate spring as the outer horizontal plate spring moves back and forth from the open end of the outer tube.

In addition, the present disclosure provides a manipulator in which the outer horizontal plate spring and the horizontal plate spring have a smooth surface with no irregularities adjacent to each other in an extending direction at a central portion in the extending direction.

According to this manipulator, it becomes difficult for body fluid such as blood to adhere to the outer horizontal plate spring or the horizontal plate spring. In addition, the attached body fluid such as blood can be easily removed from the outer horizontal plate spring and the horizontal plate spring by cleaning. In addition, in particular, the outer horizontal plate spring can be smoothly moved back and forth from the open end of the outer tube.

In addition, the present disclosure provides a manipulator in which the holder includes a pair of guides that comes into contact with a curved outer surface of the curved vertical plate spring of each of the pair of flexible operating bodies.

According to this manipulator, the deformation of the curved vertical plate spring in the further curved direction is restricted by the guide that comes into contact with the curved outer surface. Accordingly, the deformation of the curved vertical plate spring in the curved direction due to the reaction force from the shaft body is restricted. As a result, a large moment can be applied to the shaft body.

In addition, the present disclosure provides a manipulator in which the holder is integrally formed of at least one of metal and resin.

According to this manipulator, the holder is integrally formed of metal or resin. That is, in the holder, the holder body and the pair of outer horizontal plate springs are integrally formed of metal or resin. In the present specification, the term "integrally formed" does not include a structure joined by a fixture (bolt, nut, grommet, eyelet, rivet), a caulking portion, adhesives, or the like. In the holder, forming integrally includes, for example, a manufacturing method in which unnecessary parts are removed by cutting, grinding, or electrical discharge machining (such as wire cut electric discharge machining), or laser processing of a tube material as a material to obtain a structure having a desired shape (the holder). In addition, as an integral formation, for example, the holder having a desired shape may be obtained by filling a mold with a resin material. In this case, two-color molding and insert molding are included. Therefore, "integrally" is synonymous with "integral" in terms of "being in a relationship that cannot be divided into one", but is not required to be made of a single material. Therefore, the integrally formed holder does not have a fixing portion joined using another member.

In addition, the present disclosure provides a manipulator in which the holder is divided into a first holder that supports one end of the pin and is integrally formed with one of the pair of outer horizontal plate springs and a second holder that supports the other end of the pin and is integrally formed with the other of the pair of outer horizontal plate springs.

According to this manipulator, the divided first holder and second holder have no part for integrally linking both (that is, a guide). In the divided first holder and second holder, it is possible to prevent friction caused by the curved vertical plate spring and the guide rubbing each other. Accordingly, with the divided first holder and second holder, the tilting accuracy of the operation portion can be improved, and the destruction of the curved vertical plate spring due to friction can be prevented. In addition, with the divided first holder and second holder, the manufacture becomes easier as compared with the integral holder linked by the guide. Furthermore, compared to the integral holder, the friction with the guide is eliminated, so that the tilting of the operation portion can be made smooth.

In addition, the present disclosure provides a manipulator in which a fixing portion that fixes both ends of the pin to each of the first holder and the second holder is formed in a circular shape having a diameter larger than approximately half of a width dimension orthogonal to an extending direction of the outer horizontal plate spring.

According to this manipulator, the fixing portion is formed in a circular shape having a diameter larger than half of the width dimension of the outer horizontal plate spring. Therefore, the first holder and the second holder can restrict the pin from coming off from the holding plate with high strength compared to the structure in which both ends of the pin are fixed by a fixing portion having a diameter smaller than approximately half or the half of the width dimension of the outer horizontal plate spring. Accordingly, the fixing strength of the pin can be enhanced in response to the stress acting on the fixing portion of both ends of the pin and the holding plate which is increased by eliminating the guide.

### ADVANTAGEOUS EFFECTS OF INVENTION

According to the present disclosure, a wire can be eliminated, size, weight, and cost can be reduced with a small number of components, and an easy approach to the target lesion is possible.

### BRIEF DESCRIPTION OF DRAWINGS

FIG. 1 is an external perspective view of a manipulator of the present embodiment.
FIG. 2 is an enlarged perspective view of a main part of a tip end portion of the manipulator shown in FIG. 1.
FIG. 3 is a plan view in which a part of the tip end portion shown in FIG. 2 is cut away.
FIG. 4 is a side view in which a part of the tip end portion shown in FIG. 2 is cut away.
FIG. 5 is a perspective view in which the tip end portion shown in FIG. 2 is rotated 90 degrees clockwise around a tube axis.
FIG. 6 is a perspective view in which an outer tube of the tip end portion shown in FIG. 5 is omitted.
FIG. 7 is a perspective view in which a holder of the tip end portion shown in FIG. 6 is omitted.
FIG. 8 is a perspective view of one flexible operating body shown in FIG. 7.
FIG. 9 is a plan view of a first flexible operating body in which a pin shown in FIG. 8 is omitted.
FIG. 10 is a perspective view of the holder shown in FIG. 5.
FIG. 11 is an explanatory view of a displacement direction of an operation portion.
FIG. 12 is an operation explanatory diagram showing the displacement direction of the operation portion by a pair of horizontal plate springs.
FIG. 13 is an operation explanatory diagram showing the displacement direction of the operation portion by a pair of outer horizontal plate springs.
FIG. 14 is an enlarged perspective view of a main part of the manipulator in which the operation portion is opened.
FIG. 15 is an enlarged perspective view of the main part of the manipulator in which the operation portion is rotated in a -Y direction.
FIG. 16 is an enlarged perspective view of the main part of the manipulator in which the operation portion is displaced in an X direction.
FIG. 17 is an enlarged perspective view of the main part of the manipulator in which the operation portion is opened and displaced in the X direction.
FIG. 18 is an enlarged perspective view of a main part of a tip end portion of a manipulator according to a modification example.
FIG. 19 is a side sectional view of the manipulator shown in FIG. 18.

### DESCRIPTION OF EMBODIMENTS

Hereinafter, an embodiment in which a manipulator according to the present disclosure (hereinafter referred to as the embodiment) specifically disclosed will be described in detail with reference to the drawings as appropriate. Detailed description more than necessary may be omitted in some cases. For example, detailed descriptions of already well-known matters and redundant descriptions on substantially the same configuration may be omitted in some cases. This is to avoid the unnecessary redundancy of the following description and to make it easy to understand for those skilled in the art. In addition, the attached drawings and the following description are provided to enable those skilled in the art to fully understand the present disclosure, and are not intended to limit the subject matter described in the claims. In addition, in the following embodiment, the manipulator according to the present disclosure will be described by exemplifying a manipulator used for laparoscopic surgery or neurosurgery in a minimally invasive surgical procedure, for example.

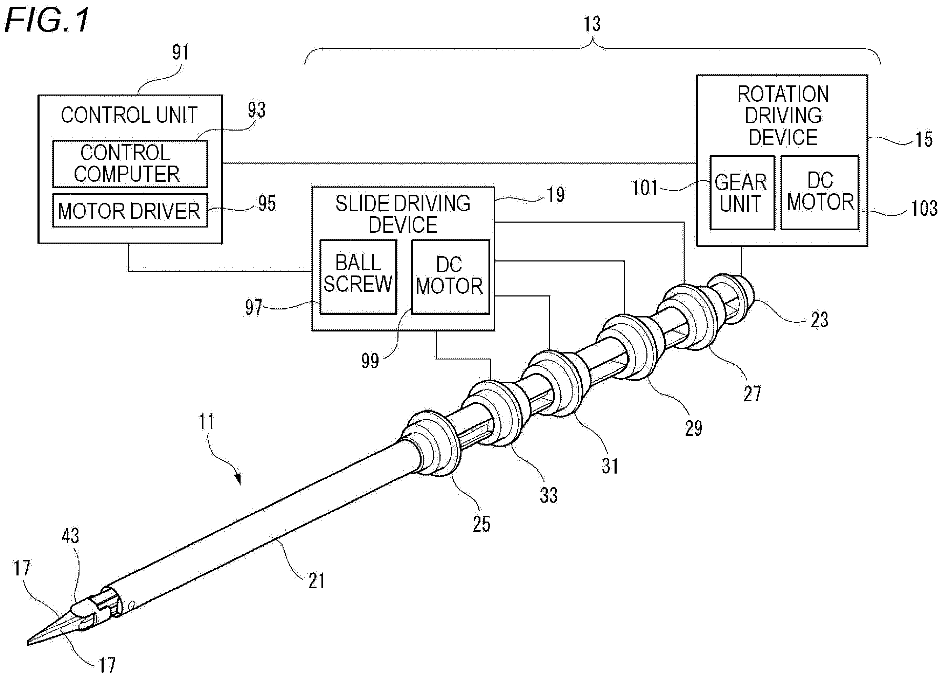

FIG. 1 is an external perspective view of a manipulator 11 of the present embodiment.

The manipulator 11 of the present embodiment is attached to a manipulator driving unit 13. The manipulator driving unit 13 is fixed to a link unit (not shown). The manipulator 11 has a tip end side inserted into a trocar (not shown) fixed to the link unit. The manipulator 11 moves with multiple degrees of freedom around one rotation center by the link unit driven by a link driving unit (not shown) together with the trocar.

The manipulator 11 rotates on inside of the trocar by a rotation driving device 15 provided in the manipulator driving unit 13. In addition, an operation portion 17 provided on the tip end side of the manipulator 11 is operated with multiple degrees of freedom by a slide driving device 19 provided in the manipulator driving unit 13. At least one operation portion 17 constitutes an end effector. The end effector means an actual working part of a surgical instrument and includes, for example, a clamp, a capturing device, scissors, a stapler, and a needle holder. A pair of operation portions 17 can be used as an end effector such as clamps, capturing devices, scissors, and staplers. One operation portion 17 can be used as an end effector such as a needle holder. In the present embodiment, the end effector can be used as a capturing device that can be configured of one operation portion 17, but for the sake of more specific description, a capturing device configured of a pair of operation portion 17 will be exemplified in the description.

In standard laparoscopic surgery, for example, the manipulator 11 is inserted through the above-described trocar that has passed through a small (approximately 1/2 inch) incision in the abdomen. A surgeon manipulates the end effector disposed at the internal surgical site via the manipulator 11 from the outside of the abdomen. The surgeon observes the procedure with a monitor (not shown) that displays an image of the surgical site taken from the laparoscope by the endoscope (not shown). A similar endoscopic procedure can be employed to arthroscopy, retroperitoneoscopy, pelviscopy, nephroscopy, cystoscopy, cisternoscopy, sinoscopy, hysteroscopy, urethroscopy, and the like.

The manipulator 11 has an outer tube 21. The outer tube 21 is made of, for example, a stainless steel tube, and is formed with an outer diameter of 6 mm. In the manipulator 11 applied to neurosurgery, the outer tube 21 is formed with a smaller diameter (outer diameter of approximately 4 mm or less). The outer tube 21 is formed with an outer diameter smaller than 8.5 mm in the "da Vinci surgical system" system of Non-Patent Literature 1 described above. The manipulator 11 is formed such that the distance from the tip end of the operation portion 17 to the manipulator driving unit 13 is 125 mm to 300 mm. The outer periphery of the outer tube 21 may be further covered with a sheath.

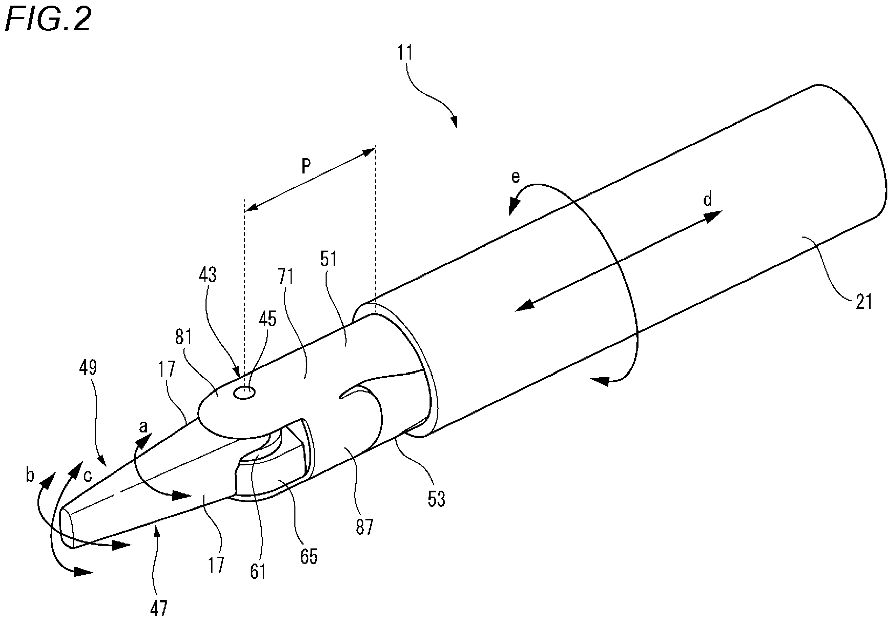

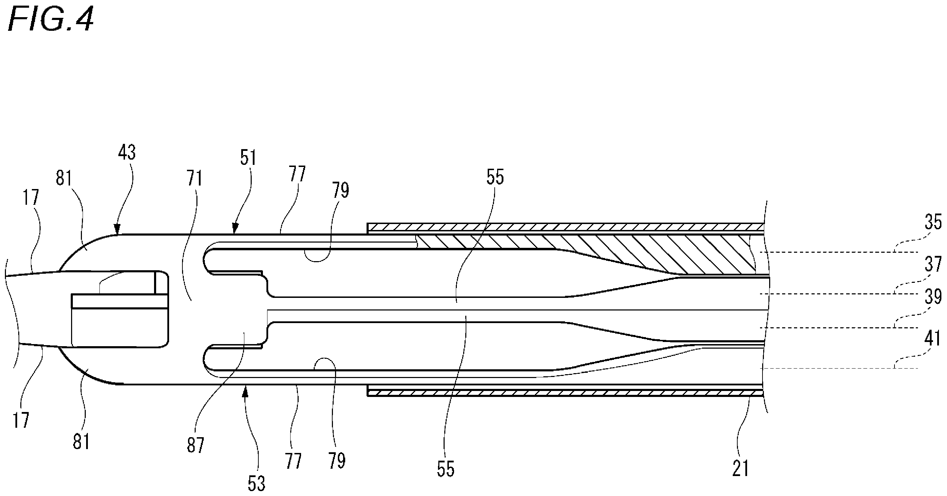

An end cap 23 is fixed to the proximal end side of the outer tube 21 in an insertion direction (that is, the side opposite to the operation portion side). The manipulator 11 integrally rotates in an e direction in FIG. 2 as the end cap 23 rotates by the rotation driving device 15 provided in the manipulator driving unit 13. A stopper 25 is fixed to the front side of the end cap 23 in the insertion direction (that is, operation portion side). The stopper 25 engages with the manipulator driving unit 13 and restricts the movement of the manipulator 11 in a longitudinal direction. Between the end cap 23 and the stopper 25, four sliders of a first outer slider 27, a first slider 29, a second slider 31, and a second outer slider 33 from the proximal end side (that is, the side opposite to the operation portion side) are respectively provided to be freely movable in the longitudinal direction of the outer tube 21. These four sliders (that is, the first outer slider 27, the first slider 29, the second slider 31, and the second outer slider 33) are inserted into the outer tube 21, and are respectively linked to a first outer slider shaft 35, a first slider shaft 37, a second slider shaft 39, and a second outer slider shaft 41 (see FIG. 4) elongated toward the tip end (that is, the operation portion side).

As a ball screw of the slide driving device 19 moves, each of the first outer slider shaft 35, the first slider shaft 37, the second slider shaft 39, and the second outer slider shaft 41 is moved (that is, pushed and pulled) in the longitudinal direction being independent from each other by the movement of the first outer slider 27, the first slider 29, the second slider 31, and the second outer slider 33. The end effector provided at the tip end of the outer tube 21 (that is, the operation portion side) operates with multiple degrees of freedom by these four shafts.

FIG. 2 is an enlarged perspective view of a main part of a tip end portion of the manipulator 11 shown in FIG. 1.

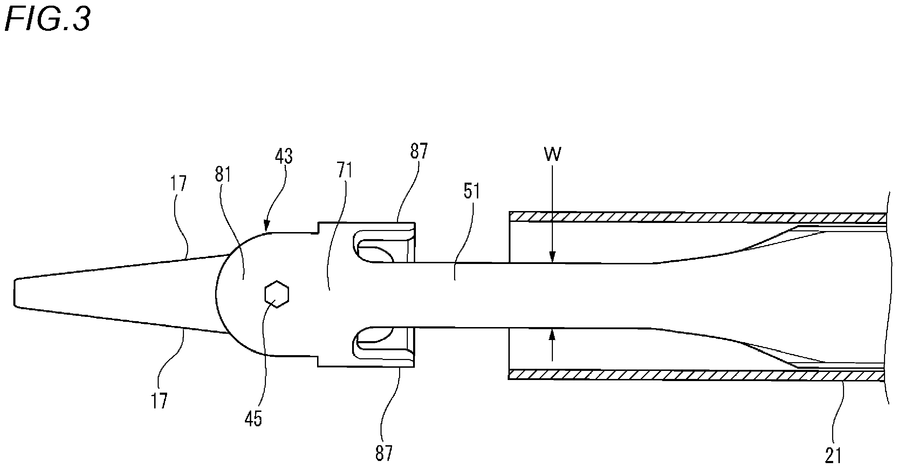

FIG. 3 is a plan view in which a part of the tip end portion shown in FIG. 2 is cut away.

FIG. 4 is a side view in which a part of the tip end portion shown in FIG. 2 is cut away.

The tip end portion of the manipulator 11 is configured with a pair of flexible operating bodies, a holder 43 integrally formed with the pair of outer horizontal plate springs, and a pin 45. In the present embodiment, the pair of flexible operating bodies has a first flexible operating body 47 and a second flexible operating body 49. In addition, the pair of outer horizontal plate springs has a first outer horizontal plate spring 51 and a second outer horizontal plate spring 53 shown in FIG. 4. The first outer horizontal plate spring 51, the first flexible operating body 47, the second flexible operating body 49, and the second outer horizontal plate spring 53 are formed so as to be elongated along the longitudinal direction with respect to the outer tube 21, and one end in the longitudinal direction extends from the inside of the outer tube 21. In the present specification, the direction on one end side is the direction on the tip end side (that is, the operation portion 17 side) of the manipulator 11. The first outer horizontal plate spring 51, the first flexible operating body 47, the second flexible operating body 49, and the second outer horizontal plate spring 53 are accommodated in the outer tube 21 from the other end in the longitudinal direction to the middle in the longitudinal direction.

As shown in FIG. 4, inside the outer tube 21, the first outer horizontal plate spring 51 is connected to the first outer slider shaft 35. The first flexible operating body 47 is connected to the first slider shaft 37. The second flexible operating body 49 is connected to the second slider shaft 39. The second outer horizontal plate spring 53 is connected to the second outer slider shaft 41.

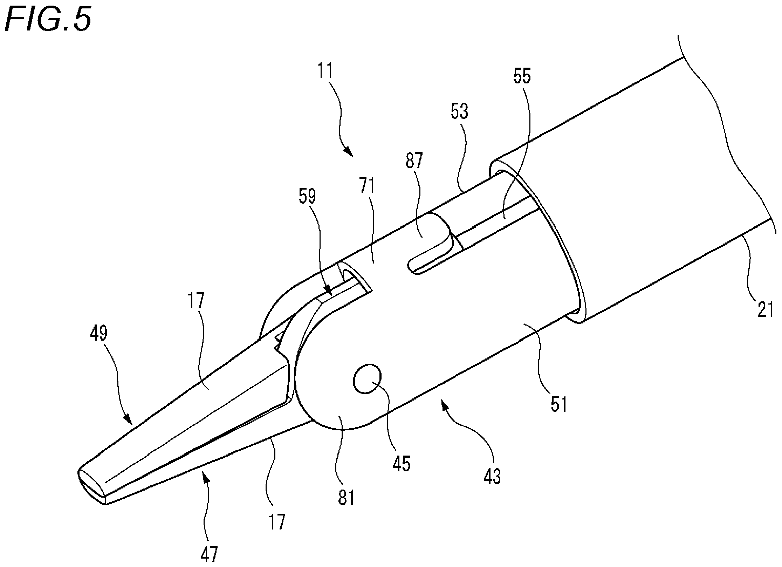

FIG. 5 is a perspective view in which the tip end portion shown in FIG. 2 is rotated 90 degrees clockwise around a tube axis.

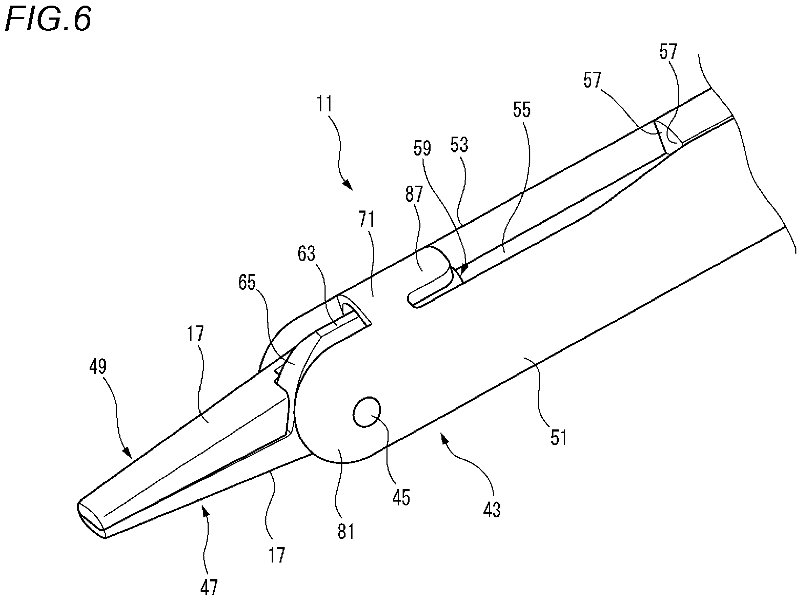

FIG. 6 is a perspective view in which the outer tube 21 of the tip end portion shown in FIG. 5 is omitted.

As shown in FIG. 5, the first outer horizontal plate spring 51 and the second outer horizontal plate spring 53, which are a pair of outer horizontal plate springs, are disposed inside the outer tube 21 with a part in the extending direction in contact with the inner wall surface of the outer tube 21. The holder 43 is supported on the outer tube 21 by the first outer horizontal plate spring 51 and the second outer horizontal plate spring 53. As shown in FIG. 6, the holder 43 supports both ends of the pin 45. The pin 45 is supported by the holder 43 with its axial movement restricted. The pin 45 passes through the first flexible operating body 47 and the second flexible operating body 49.

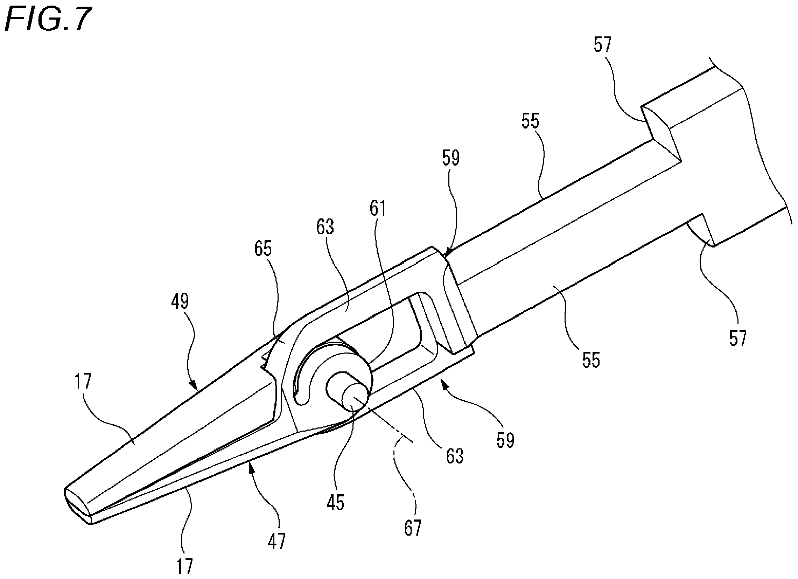

FIG. 7 is a perspective view in which the holder 43 of the tip end portion shown in FIG. 6 is omitted.

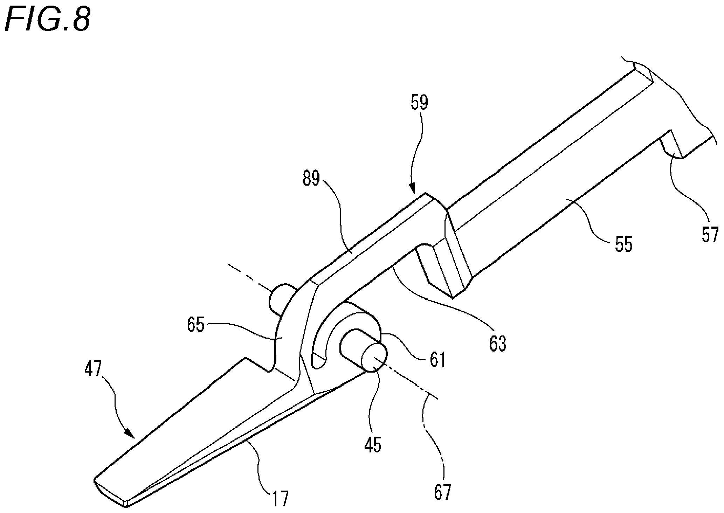

FIG. 8 is a perspective view of one flexible operating body shown in FIG. 7.

The manipulator 11 has a configuration in which the operation portions 17 of the first flexible operating body 47 and the second flexible operating body 49 are operated with multiple degrees of freedom by deforming a part of the first flexible operating body 47 and a part of the second flexible operating body 49 based on push and pull of a plate spring (such as a horizontal plate spring 55 described later, the first outer horizontal plate spring 51, the second outer horizontal plate spring 53, or a combination thereof). In the present embodiment, since the end effector will be described as a capturing device, a pair of first flexible operating body 47 and second flexible operating body 49 is used as a flexible operating body.

The pair of first flexible operating body 47 and second flexible operating body 49 has a widened portion 57 formed at one end portion of the horizontal plate spring 55 on the opposite side of the operation portion 17. The widened portion 57 comes into contact with the outer tube 21 on both sides in the plate width direction. Accordingly, the first flexible operating body 47 and the second flexible operating body 49 are restricted from moving in the plate width direction of the horizontal plate spring 55 in the outer tube.

The first flexible operating body 47 and the second flexible operating body 49 are used by inverting the same object upside down. Therefore, hereinafter, the first flexible operating body 47 will be described as a representative example of a flexible operating body.

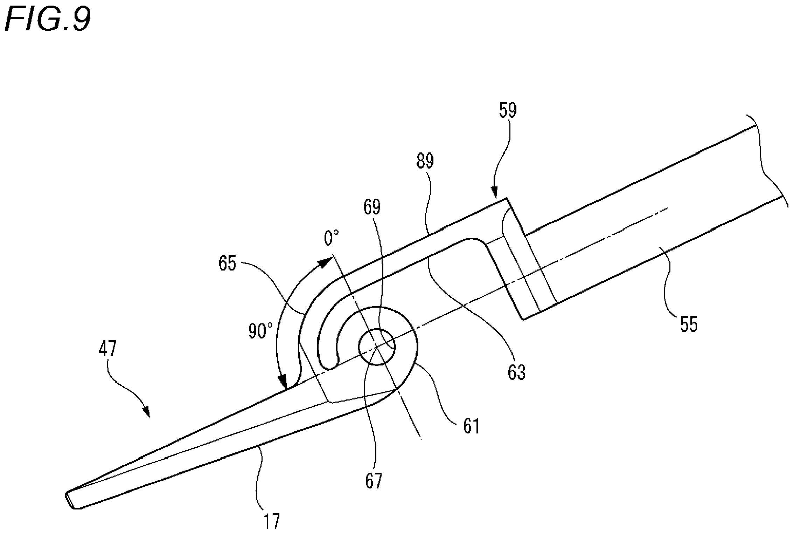

FIG. 9 is a plan view of the first flexible operating body 47 in which the pin 45 shown in FIG. 8 is omitted.

As shown in FIG. 9, in the first flexible operating body 47, the horizontal plate spring 55, a curved vertical plate spring 59, a shaft body 61, and the operation portion 17 are integrally formed. As the material of the first flexible operating body 47, for example, Ni-Ti (nickel titanium) which is excellent in bio compatibility, a wide elastic range, and corrosion resistance, is used. The horizontal plate spring 55 is formed in a long rectangular shape in the longitudinal direction of the outer tube 21. The horizontal plate spring 55 has a plate spring shape, and by employing such a shape, the unidirectionality of deformation increases, the twist is suppressed, and it is possible to operate the manipulator 11 with higher positioning accuracy. That is, the deformation direction of the first flexible operating body 47 can be controlled by the shape of the horizontal plate spring 55, and the positioning of the end effector is possible. For example, the horizontal plate spring 55 can be formed with a plate thickness of 0.4 mm. The horizontal plate spring 55 can be formed with a plate thickness of 0.25 mm when applied to neurosurgery.

The curved vertical plate spring 59 stands upright having a plate surface perpendicular to a plate surface of the horizontal plate spring 55, and the proximal end of the curved vertical plate spring 59 is connected to the one end of the horizontal plate spring 55 in the longitudinal direction. The curved vertical plate spring 59 protrudes to one side in a plate width direction of the horizontal plate spring 55 (for example, in a direction toward an upper left side of the paper surface of FIG. 9), and extends along the longitudinal direction of the horizontal plate spring 55. In the curved vertical plate spring 59, the tip end in the extending direction is an R portion 65, as an example of a bent portion in which the tip end portion in the extending direction bends in a direction opposite to the protruding direction of a bent portion 63. The curved vertical plate spring 59 is provided with the R portion 65 at a connecting part with the shaft body 61, and accordingly, efficient power transmission can be performed during the deformation operation of the first flexible operating body 47 to make it easy to perform the deformation operation. For example, the curved vertical plate spring 59 can be formed with a plate thickness of the bent portion 63 to be 0.3 mm. The curved vertical plate spring 59 can be formed with a plate thickness of 0.2 mm when applied to neurosurgery.

In the first flexible operating body 47, the tip end of the R portion 65 of the curved vertical plate spring 59 is connected to the outer periphery of the shaft body 61. The shaft body 61 is supported by a rotation center 67 perpendicular to the plate surface of the horizontal plate spring 55 and is rotatable. More specifically, a pin hole 69 is bored in the shaft body 61. The pin 45 (see FIG. 8) is inserted into the pin hole 69 coaxially to the rotation center 67. Both ends of the pin 45 are supported by the holder 43. The holder 43 is supported on the outer tube 21 by the pair of outer horizontal plate springs (that is, the first outer horizontal plate spring 51 and the second outer horizontal plate spring 53).

In the shaft body 61, when a radial position on the side on which the bent portion 63 protrudes from the rotation center 67 is 0 degrees, the R portion 65 is connected to the outer periphery at a position of approximately 90 degrees counterclockwise in FIG. 9. The R portion 65 is connected so as to wound the shaft body 61 at a position 90 degrees from a position of 0 degrees of the outer periphery. It is preferable that the R portion 65 is connected to the shaft body 61, for example, in the range from the position of 0 degrees to 120 degrees of the outer periphery, from the view point of space efficiency.

The operation portion 17 is provided to protrude to the outside of rotation radius (that is, in the radial direction) at the outer periphery of the shaft body 61. In the present embodiment, the operation portion 17 is connected to the outer periphery of the shaft body 61 of which the proximal end substantially coincides with the connection point of the R portion 65. The operation portion 17 configured with capturing device has a plate width along the rotation center 67 and is formed in a substantially pyramid shape in which the plate width and the plate thickness gradually decrease toward the protrusion tip end.

In addition, as shown in FIG. 7, in the manipulator 11, the first flexible operating body 47 on one side and the second flexible operating body 49 on the other side are overlapped such that the protruding direction of the bent portion 63 is opposite to the protruding direction of the bent portion 63. The first flexible operating body 47 and the second flexible operating body 49 are assembled such that the pair of shaft bodies 61 overlaps each other in the direction along the rotation center 67 and the pair of shaft bodies 61 and the operation portion 17 have the same height in the direction along the rotation center 67.

The first flexible operating body 47 and the second flexible operating body 49 are linked to each other to be rotatable about the same rotation center 67 by the pin 45 that penetrates both shaft bodies 61. The first flexible operating body 47 and the second flexible operating body 49 which are integrally assembled are accommodated in the outer tube 21 from the other end in the longitudinal direction to the middle part in the longitudinal direction of the horizontal plate spring 55 of each of the first flexible operating body 47 and the second flexible operating body 49.

The first flexible operating body 47 and the second flexible operating body 49 are disposed such that each of the operation portions 17 intersects each other in a state where the shaft bodies overlaps each other. Therefore, the operation portion 17 of the first flexible operating body 47 shown in FIG. 9 becomes the operation portion 17 on the lower right side of the paper surface of FIG. 7 when the two operation portions 17 shown in FIG. 7 face the lower side of the paper surface. In addition, similarly, the operation portion 17 of the second flexible operating body 49 becomes the operation portion 17 on the upper left side of the paper surface shown in FIG. 7 when the two operation portions 17 shown in FIG. 7 face the lower side of the paper surface. Therefore, the pair of operation portions 17 is rotated in a direction of approaching each other (that is, gripping) by pulling the first flexible operating body 47 and the second flexible operating body 49 at the same time. That is, the first flexible operating body 47 and the second flexible operating body 49 convert the force in the linear direction into a moment about the pin 45 by the curved vertical plate spring 59. Therefore, the first flexible operating body 47 and the second flexible operating body 49 can avoid buckling that occurs in the curved vertical plate spring 59 and can obtain a large gripping force, as compared with the case of pushing.

In addition, in the first flexible operating body 47, the rotation center 67 of the shaft body 61 is disposed on an extension line of the axis line which passes through the center in the plate width direction of the horizontal plate spring 55. That is, the first flexible operating body 47 has a shape in which only the curved vertical plate spring 59 bulges from one side from the horizontal plate spring 55, the shaft body 61, and the operation portion 17 which are disposed substantially linearly.

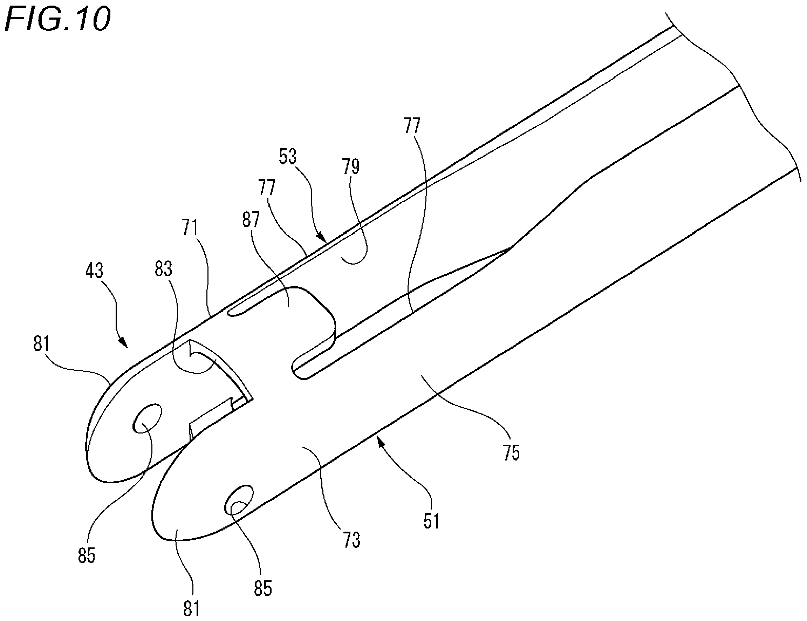

FIG. 10 is a perspective view of the holder 43 shown in FIG. 5.

The manipulator 11 includes the holder 43 that supports both ends of the pin 45 that passes through the pair of overlapped shaft bodies 61. In the holder 43, the pair of outer horizontal plate springs (the first outer horizontal plate spring 51 and the second outer horizontal plate spring 53) which has the horizontal plate springs 55 of the pair of flexible operating bodies (the first flexible operating body 47 and the second flexible operating body 49) to be interposed in parallel between the holder 43 and extends in the same direction as the horizontal plate spring 55 is integrally formed.

The holder 43 is integrally formed of at least one of metal and resin. In the present embodiment, the holder 43 is made of, for example, Ni-Ti (nickel titanium) which is excellent in biocompatibility, a wide elastic range, and corrosion resistance similar to the first flexible operating body 47. The holder 43 can be manufactured by wire cut electric discharge machining of a nickel titanium tube material. When the manipulator 11 is applied to neurosurgery, a tube material having an outer diameter of 3 mm and an inner diameter of 1.4 mm is used as the material of the holder 43.

The pair of outer horizontal plate springs is formed from this tube material by wire cut electric discharge machining. Accordingly, the pair of outer horizontal plate springs (the first outer horizontal plate spring 51 and the second outer horizontal plate spring 53) extends from a cylindrical outer peripheral surface 73 of a holder body 71 of the holder 43 in the direction along a generatrix and has an arc-shaped outer peripheral surface 75 having the same radius of curvature as the cylindrical outer peripheral surface 73.

The first outer horizontal plate spring 51, the second outer horizontal plate spring 53 are formed by inverting the same shape upside down. Hereinafter, the first outer horizontal plate spring 51 will be described as a representative example of an outer horizontal plate spring.

As shown in FIGS. 4 and 10, in the first outer horizontal plate spring 51, a flexible portion 77 that is thinner and narrower than both ends of the outer horizontal plate spring in the longitudinal direction is formed at the central portion in the extending direction. By forming the flexible portion 77 in the first outer horizontal plate spring 51, the holder 43 concentrates stress on the flexible portion 77 to make the first outer horizontal plate spring 51 elastically deformable. The flexible portion 77 is formed with a length in the extending direction of approximately 7 mm and a width of approximately 1.4 mm.

The first outer horizontal plate spring 51 has an opposing flat surface 79 on the opposite side of the arc-shaped outer peripheral surface 75. In the first outer horizontal plate spring 51, by employing such a shape, the unidirectionality of deformation increases, the twist is suppressed, and it is possible to operate the manipulator 11 with higher positioning accuracy. That is, the deformation direction of the first flexible operating body 47 can be controlled by the shape of the first outer horizontal plate spring 51, and the positioning of the end effector is possible.

The first outer horizontal plate spring 51 and the above-described horizontal plate spring 55 have a smooth surface with no irregularities adjacent to each other in the extending direction at the central portion in the extending direction. Accordingly, the manipulator 11 makes it difficult for body fluid such as blood to adhere to the first outer horizontal plate spring 51 and the horizontal plate spring 55 exposed from the outer tube 21. In addition, the first outer horizontal plate spring 51 and the horizontal plate spring 55 having a smooth surface can be satisfactorily cleaned.

The holder 43 has a pair of parallel holding plates 81 that are separated from each other. One holding plate 81 is formed in a semi-cylindrical shape with a tip end curved surface formed by cutting a rotating body obtained by rotating an ellipse around a major axis along the major axis. Both of the pair of holding plates 81 which are separated from each other are connected by each of the side plates 83. Accordingly, the holder 43 has an internal space of a rectangular hole when viewed from the front. Both ends of the above-described pin 45 are fixed to pin support holes 85 bored in the pair of holding plate 81. The holder 43 accommodates the shaft body 61 and the curved vertical plate spring 59 in this internal space.

In addition, in the holder 43, a pair of parallel guides 87 is formed so as to protrude from the respective side plates 83 toward the outer tube 21. The pair of guides 87 comes into contact with a curved outer surface 89 (see FIG. 9) of each of the curved vertical plate spring 59 in the pair of first flexible operating body 47 and second flexible operating body 49. As the guide 87 comes into contact with the curved outer surface 89 of the curved vertical plate spring 59 from the outside, the deformation operation which bulges out of the curved vertical plate spring 59 can be restricted so that it is possible to effectively transmit the power to the R portion 65 and the deformation operation can be easily performed.

Next, a control unit 91 that controls the slide driving device 19 and the rotation driving device 15 of the manipulator 11 will be described.

The operation of the manipulator 11 is controlled by the control unit 91 shown in FIG. 1. The control unit 91 has a control computer 93 and a motor driver 95. The control unit 91 controls the operation of the slide driving device 19 and the rotation driving device 15. The slide driving device 19 includes a ball screw 97 and a DC motor 99. The slide driving device 19 controls each of the positions of four sliders of the first outer slider 27, the first slider 29, the second slider 31, and the second outer slider 33 in the longitudinal direction of the outer tube 21 shown in FIG. 1. The slide driving device 19 operates based on an instruction from the control unit 91.

The rotation driving device 15 includes a gear unit 101 and a DC motor 103. The rotation driving device 15 controls the rotation direction and the rotation angle of the end cap 23 at the rear end of the outer tube 21 shown in FIG. 1 in the longitudinal direction. The rotation driving device 15 operates based on an instruction from the control unit 91.

Next, the operation of the manipulator 11 will be described.

In the manipulator 11, the operation portion 17 operates with multiple degrees of freedom with respect to the outer tube 21. That is, the pair of operation portions 17 moves in a direction (an arrow a direction in FIG. 2) in which the pair of operation portions 17 moves close to and away from each other (such as opening and closing). The manipulator 11 can rotate in the forward and reverse directions (rotate in an arrow b direction in FIG. 2) around the rotation center 67 of the shaft body 61 while the pair of operation portions 17 is closed. The holder 43 can be displaced in the bending direction of the outer horizontal plate spring (an arrow c direction in FIG. 2). The holder 43 can be moved back and forth in the direction along the central axis of the outer tube 21 (an arrow d direction in FIG. 2). The manipulator 11 can further rotate the holder 43 and the outer tube 21 integrally (an arrow e direction in FIG. 2) via the end cap 23 by the rotation driving device 15.

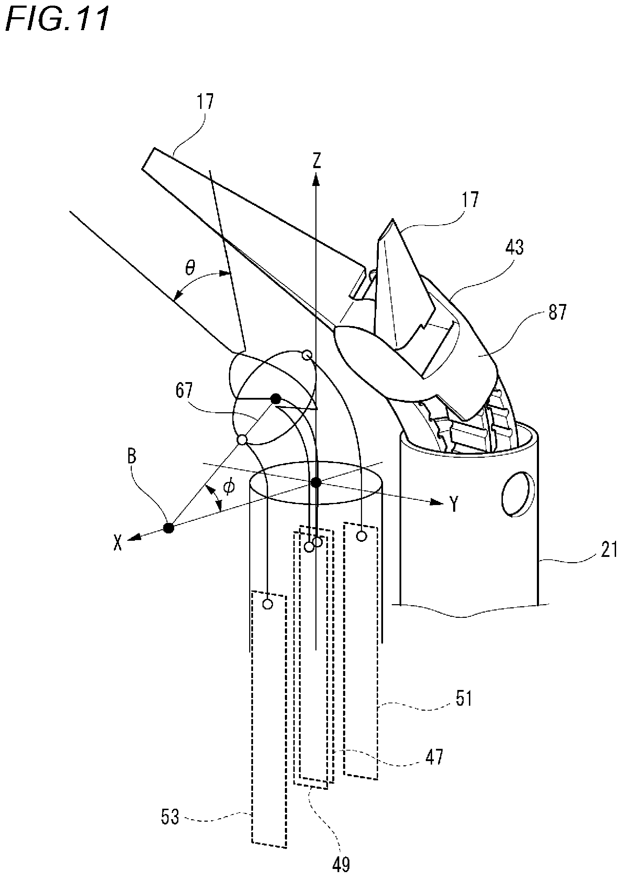

FIG. 11 is an explanatory view of a displacement direction of the operation portion 17.

Here, in order to make it easy to describe the operation at the tip end part of the manipulator 11, the direction of operation is defied. The tip end surface of the outer tube 21 is a plane orthogonal to the central axis of the outer tube 21 and has a circular shape centered on the central axis. At this time, the central axis of the outer tube 21 is "Z axis". In the manipulator 11 in a state in which the holder 43 is not displaced (a state shown in FIG. 2), the axis which passes through the Z axis on the surface including the tip end surface of the outer tube 21 and has the same direction as that of the rotation center 67 of the pin 45 is defined as an "X axis". The axis which passes through the Z axis on the surface including the tip end surface of the outer tube 21 and is orthogonal to the X axis is defined as a "Y axis". A narrow angle of the pair of operation portions 17 opened by rotating the rotation center 67 is set to " 0". When the plate spring is bent in the X axis direction, and the holder 43 is displaced (tilted), an angle formed between the rotation center 67 of the pin 45 and the X axis is set to "φ". At this time, a point at which the rotation center 67 of the pin 45 intersects with the X axis is a bending center B.

The manipulator 11 tilts the end effector with the distance from the tip end surface of the outer tube 21 to the pin 45 as a bendable range. The distance P (see FIG. 2) from the tip end surface of the outer tube 21 to the rotation center 67 of the pin 45 is set to be, for example, 7.5 mm. The distance P is shorter than the equivalent distance of 9 mm in the above-described "da Vinci surgical system". Accordingly, it is possible to reduce the bending radius when the end effector tilts. This contributes to the ease of a smooth approach to the narrow site (that is, target lesion), for example, in laparoscopic surgery. The distance P is set to 4.85 mm in the manipulator 11 applied to neurosurgery.

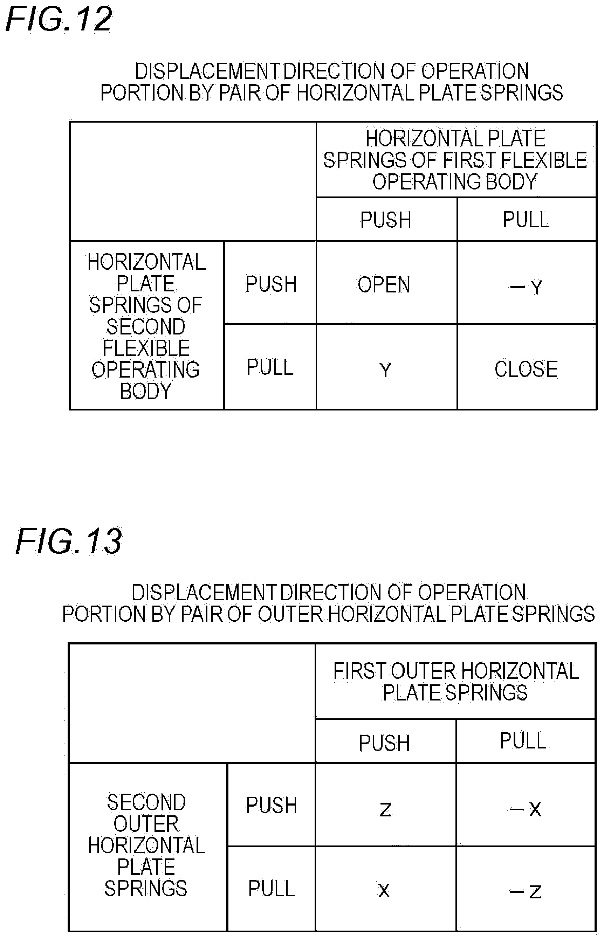

FIG. 12 is an operation explanatory diagram showing the displacement direction of the operation portion 17 by the pair of horizontal plate springs 55.

FIG. 13 is an operation explanatory diagram showing the displacement direction of the operation portion 17 by the pair of outer horizontal plate springs.

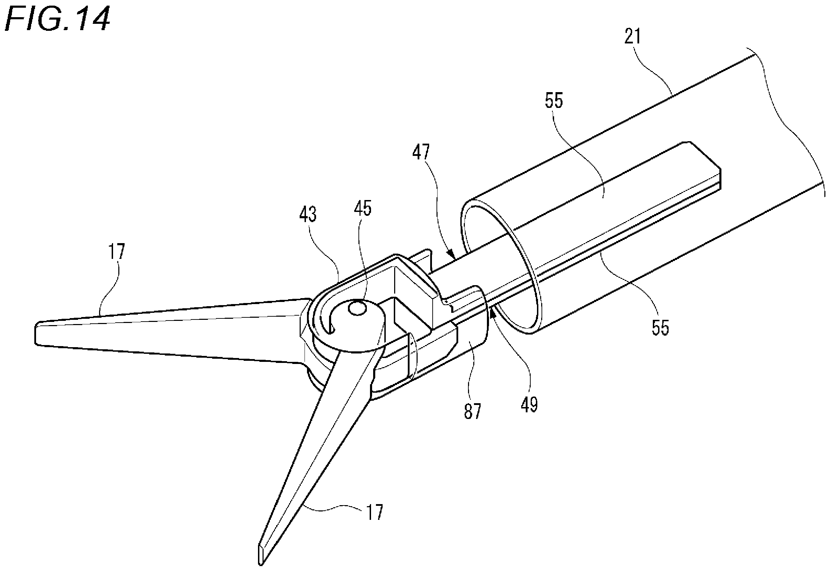

FIG. 14 is an enlarged perspective view of a main part of the manipulator 11 in which the operation portion 17 is opened.

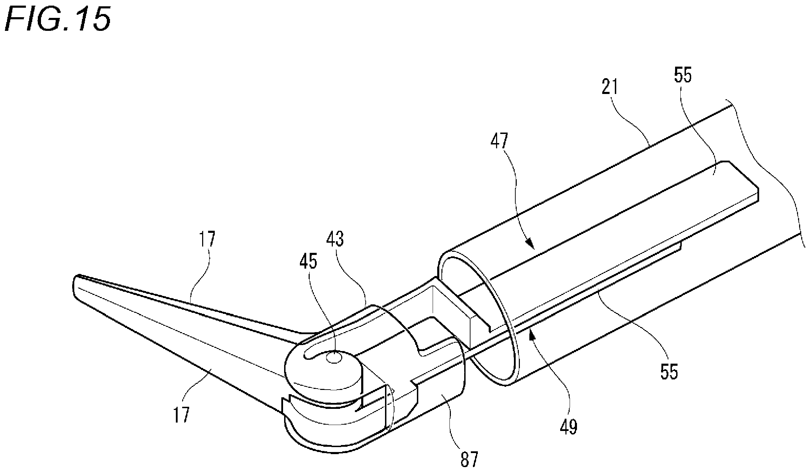

FIG. 15 is an enlarged perspective view of the main part of the manipulator 11 in which the operation portion 17 is rotated in a -Y direction.

In the manipulator 11, as shown in FIG. 12, when the horizontal plate spring 55 of the first flexible operating body 47 and the horizontal plate spring 55 of the second flexible operating body 49 are pushed at the same time (that is, pushed to the operation portion side), the pair of shaft bodies 61 rotates in the reverse direction. That is, the pair of operation portions 17 opens as shown in FIG. 14. In addition, in the manipulator 11, when the horizontal plate spring 55 of the first flexible operating body 47 and the horizontal plate spring 55 of the second flexible operating body 49 are pulled at the same time (that is, pulled to the opposite side of the operation portion side), the pair of shaft bodies 61 rotates in the direction reverse to that when the pair of shaft bodies 61 is opened. That is, as shown in FIG. 2, the pair of operation portions 17 is closed.

In the manipulator 11, when the horizontal plate spring 55 of the first flexible operating body 47 is pulled and the horizontal plate spring 55 of the second flexible operating body 49 is pushed, as shown in FIG. 15, the pair of operation portions 17 rotates clockwise in FIG. 2 about the rotation center 67 while being closed, and tilts to the -Y axis side. In addition, in the manipulator 11, when the horizontal plate spring 55 of the first flexible operating body 47 is pushed and the horizontal plate spring 55 of the second flexible operating body 49 is pulled, the pair of operation portions 17 rotates counterclockwise in FIG. 2 around the rotation center 67 while being closed, and tilts to the Y axis side.

In the manipulator 11, as shown in FIG. 13, when the first outer horizontal plate spring 51 and the second outer horizontal plate spring 53 are pushed at the same time, the pair of operation portions 17 advances in the Z direction. In addition, in the manipulator 11, when the first outer horizontal plate spring 51 and the second outer horizontal plate spring 53 are pulled at the same time, the pair of operation portions 17 retreats in the -Z direction. During the forward and backward movements of the first outer horizontal plate spring 51 and the second outer horizontal plate spring 53, the first flexible operating body 47 and the second flexible operating body 49 are driven or pushed and pulled at the same time.

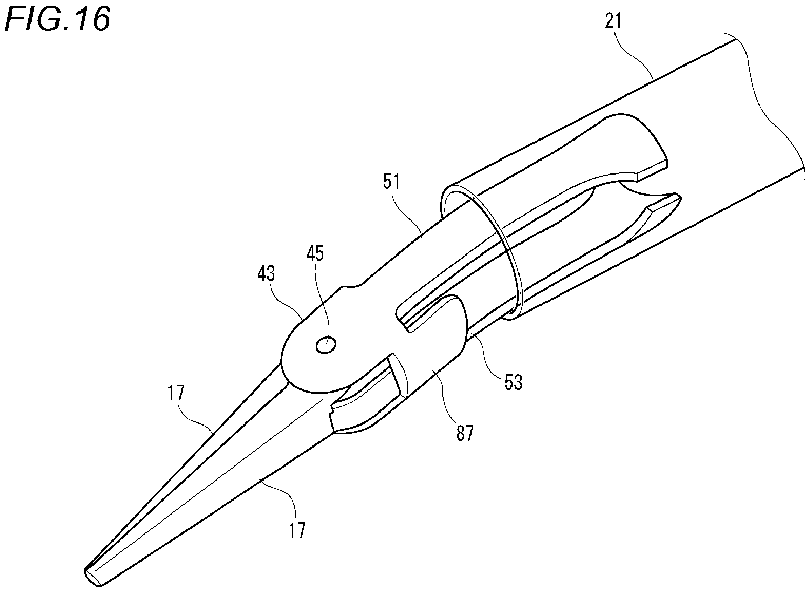

FIG. 16 is an enlarged perspective view of the main part of the manipulator 11 in which the operation portion 17 is displaced in the X direction.

In the manipulator 11, when the first outer horizontal plate spring 51 is pushed and the second outer horizontal plate spring 53 is pulled, as shown in FIG. 16, the pair of operation portions 17 tilts to the X axis side. In addition, in the manipulator 11, when the first outer horizontal plate spring 51 is pulled and the second outer horizontal plate spring 53 is pushed, the pair of operation portions 17 tilts to the -X axis side.

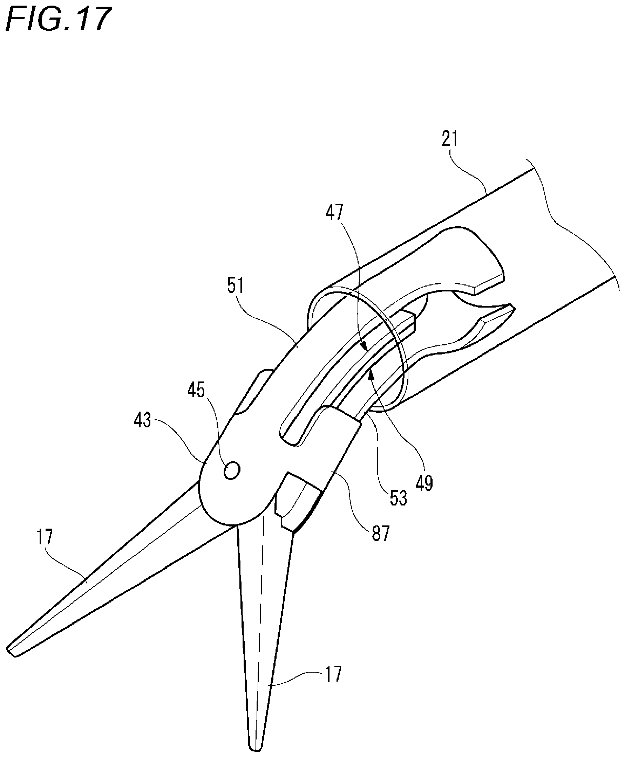

FIG. 17 is an enlarged perspective view of the main part of the manipulator 11 in which the operation portion 17 is opened and displaced in the X direction.

The manipulator 11 can move the bending center B (see FIG. 11) by changing the pushing and pulling amounts of the first outer horizontal plate spring 51 and the second outer horizontal plate spring 53. In addition, as shown in FIG. 17, the manipulator 11 can open and close the pair of operation portions 17 by changing the pushing and pulling amounts of the first flexible operating body 47 and the second flexible operating body 49 at the same time as the movement of the bending center B.

The manipulator 11 obtains the target values of feeding amounts of the first outer slider 27, the first slider 29, the second slider 31, and the second outer slider 33 of the manipulator 11 with respect to the target bending angle φ by the control computer 93, transmits a control signal from the motor driver 95, drives the ball screw 97 with the DC motor 99 in the slide driving device 19, and accordingly, the tip end of the manipulator 11 (that is, the end effector, and more specifically, a pair of operation portions 17) is operated. Accordingly, as shown in FIG. 17, the manipulator 11 can open and close the pair of operation portions 17 while bending the end effector to the target bending angle φ. Furthermore, the manipulator 11 changes the pushing and pulling amounts of the first flexible operating body 47 and the second flexible operating body 49 while changing the pushing and pulling amounts of the first outer horizontal plate spring 51 and the second outer horizontal plate spring 53, and accordingly, the tip end of the end effector can be swiveled in any direction of 360 degrees around the Z axis.

Next, the operation of the configuration of the above-described manipulator 11 and the effect of the operation will be specifically described.

In the manipulator 11 of the present embodiment, the horizontal plate spring 55, the curved vertical plate spring 59, and the shaft body 61 are disposed in series in on the same plane to configure a flexible operating body (that is, the first flexible operating body 47 and the second flexible operating body 49). The curved vertical plate spring 59 is connected to the horizontal plate spring 55 in a direction in which the plate surface stands upright. That is, the horizontal plate spring 55 and the curved vertical plate spring 59 are connected to each other of which the plate surfaces are orthogonal to each other. The shaft body 61 is supported to be rotatable at the rotation center 67 perpendicular to the plate surface of the horizontal plate spring 55.

When the other end of the horizontal plate spring 55 in the longitudinal direction is pressed in the direction along the longitudinal direction, in a case where the shaft body 61 is supported so as to be immovable within the same plane as described above, the flexible operating body (that is, the first flexible operating body 47 and the second flexible operating body 49) is deformed in a direction in which the curved vertical plate spring 59 is further curved. The deformation occurs within the elastic limit of the curved vertical plate spring 59. Therefore, elongation and shrinkage (permanent distortion) do not occur in the flexible operating body. Since the horizontal plate spring 55 and the plate surface of the curved vertical plate spring 59 are orthogonal to each other, the deformation direction of the curved vertical plate spring 59 changes by 90 degrees. Accordingly, the curved vertical plate spring 59 can deform along the above-described same plane. In the curved vertical plate spring 59, most of the internal stress accumulated by the deformation is an elastic restoring force. A part of the elastic restoring force acts as a component force in a tangent direction of the outer periphery of the shaft body which generates a moment with respect to the shaft body 61. In the manipulator 11 provided with the pair of guides 87 in the holder 43, the deformation of the curved vertical plate spring 59 mainly occurs in the R portion 65.

In addition, when the other end of the horizontal plate spring 55 in the longitudinal direction is pulled in a direction along the longitudinal direction, the flexible operating body (that is, the first flexible operating body 47 and the second flexible operating body 49) is deformed in a direction of eliminating the curve (linearly approaching direction) of the curved vertical plate spring 59. In the curved vertical plate spring 59, a part of the elastic restoring force accumulated by the deformation acts as a component force in the tangent direction of the outer periphery of the shaft body which generates a moment in a direction reverse to the above-described direction, with respect to the shaft body 61. As a result, the shaft body 61 can rotate the operation portion 17 provided in the shaft body 61 in the reverse direction about the above-described rotation center 67 by pushing and pulling the horizontal plate spring 55.

Furthermore, the flexible operating body (that is, the first flexible operating body 47 and the second flexible operating body 49) allows the deformation in the direction in which the horizontal plate spring 55 is displaced to the front and rear plate surface sides (X axis side and -X axis side in FIG. 11). Accordingly, the flexible operating body does not prevent the operation portion 17 from tilting in the same direction.

In this manner, the manipulator 11 which uses the flexible operating body can eliminate the wire which is common means of the related art and operate the operation portion 17 with multiple degrees of freedom.

The flexible operating body (that is, the first flexible operating body 47 and the second flexible operating body 49) has no concern of stretching or breaking unlike a wire. Accordingly, it is possible to improve the durability, and to extremely reduce the number of replacements of the manipulator 11. Since the flexible operating body (that is, the first flexible operating body 47 and the second flexible operating body 49) does not stretch like a wire, the rotation accuracy and the positional accuracy of the operation portion 17 can be enhanced. Furthermore, since the flexible operating body (that is, the first flexible operating body 47 and the second flexible operating body 49) is a single member, a simple machine configuration can be realized, and sterilization and cleaning are extremely easy. Moreover, since the flexible operating body (that is, the first flexible operating body 47 and the second flexible operating body 49) does not need to be wound around a gear or a pulley unlike a wire, it is possible to make the attachment and detachment simple and easy between the manipulator 11 and driving means.

In addition, the flexible operating body (that is, the first flexible operating body 47 and the second flexible operating body 49) does not need to use a plurality of links and passive joints unlike a link mechanism. Accordingly, it is easy to reduce the number of components, size, and weight. According to this, it becomes easy to reduce the manufacturing cost. In addition, reduction in weight reduces the moving mass of the end effector, which makes it possible to reduce the inertial force. Accordingly, the positioning control of the end effector becomes easy and the positioning accuracy can be enhanced. In addition, since a wire, a pulley, a plurality of links, or a passive joint is not used, the bending radius when displacing the operation portion 17 together with the shaft body 61 can be reduced.

Since the protruding directions of the curved vertical plate springs 59 of the pair of flexible operating bodies are reversed to each other, the manipulator 11 pushes and pulls the other ends of the horizontal plate springs 55 in the longitudinal direction at the same time, and accordingly, each operation portion 17 can be moved close to and away from each other. That is, when the operation portion 17 is a capturing device, pinching becomes possible. When the operation portion 17 is scissors, cutting becomes possible. In the manipulator 11, by pushing or pulling the other end of the pair of horizontal plate springs 55 in the longitudinal direction at the same time in the opposite directions, the pair of operation portions 17 can be rotated about the rotation center 67 of the shaft body 61 in the same direction. That is, when the operation portion 17 is a capturing device, it is possible to rotate in forward and reverse directions while the capturing device is closed.

In addition, in the manipulator 11, the pair of curved vertical plate springs 59 protrudes in the reverse direction. In contrast, the horizontal plate springs 55 of the pair of flexible operating bodies overlap each other without deviation in the longitudinal direction and the plate width direction. In the manipulator 11, the rotation center 67 of each of the shaft bodies 61 is commonly arranged and fixed by a single pin 45 on the extension line of the axis line of the horizontal plate spring 55. Accordingly, the manipulator 11 can ensure a curved shape while suppressing the protrusion width between the protruding ends of the pair of curved vertical plate springs 59 in the curved direction to be small.

In addition, in the manipulator 11, the pair of flexible operating bodies overlaps such that the pair of horizontal plate springs 55 interposes the central axis of the outer tube 21 between the horizontal plate springs 55. In the manipulator 11, furthermore, the pair of outer horizontal plate springs (the first outer horizontal plate spring 51 and the second outer horizontal plate spring 53) is disposed on the outside of the pair of horizontal plate springs 55 so as to interpose the pair of horizontal plate springs 55 between the outer horizontal plate springs. That is, in the outer tube 21, the pair of horizontal plate springs 55 and the pair of outer horizontal plate springs are disposed in four layers. Each of the plate springs is pushed and pulled independently. One end of the pair of outer horizontal plate springs in the longitudinal direction is fixed to the holder 43. The tip end surface of the outer tube 21 and the holder 43 are disposed to be separated from each other. Therefore, between the tip end surface of the outer tube 21 and the holder 43, four layers of the overlapped pair of horizontal plate springs 55 and the pair of outer horizontal plate springs interposing the horizontal plate springs therebetween are exposed.

In the manipulator 11, when the pair of outer horizontal plate springs is pushed and pulled in the reverse direction, the pair of outer horizontal plate springs is deformed (bent) in a direction of being inclined to one outer horizontal plate spring side together, and the pair of outer horizontal plate springs is deformed (bent) in a direction of being inclined to the other outer horizontal plate spring side together. The holder 43 is displaced (that is, tilted) by the bending of the pair of outer horizontal plate springs. At this time, the pair of horizontal plate springs 55 disposed between the pair of outer horizontal plate springs also allows the bending in the same direction.

In this manner, the manipulator 11 pushes and pulls the other ends of the horizontal plate springs 55 in the longitudinal direction in the pair of flexible operating bodies to move in a direction a (see FIG. 2) of moving close to and away from the pair of operation portions 17 (1 degree of freedom).

By pushing and pulling the other ends of the horizontal plate springs 55 in the longitudinal direction in the pair of flexible operating bodies in the reverse direction, the pair of operation portions 17 can rotate in the forward and reverse directions b (see FIG. 2) about the rotation center 67 of the shaft body 61 (2 degrees of freedom).

By pushing and pulling the pair of outer horizontal plate springs in the reverse direction, the holder 43 (that is, the operation portion 17) can be displaced in the direction c (see FIG. 2) of bending of the outer horizontal plate spring (3 degrees of freedom).

In addition, by pushing and pulling all the springs of the pair of flexible operating bodies and the pair of outer horizontal plate springs in the same direction along the longitudinal direction, the pair of operation portions 17 can be moved back and forth in the direction d (see FIG. 2) along the central axis of the outer tube 21 (4 degrees of freedom).

Furthermore, as the end cap 23 rotates, the manipulator 11 rotates integrally in the e direction (see FIG. 2) around the tube axis of the outer tube 21 (5 degrees of freedom).

That is, the manipulator 11 applies the deformation of the elastic body and performs mechanical power conversion without using a wire or a link mechanism, so that the operation portion 17 can be operated with multiple degrees of freedom (specifically, 5 degrees of freedom).

In addition, in the manipulator 11, the deformation in the direction in which the curved vertical plate spring 59 is further curved is restricted by the guide 87 that comes into contact with the curved outer surface 89. Accordingly, the deformation in the curved direction of the curved vertical plate spring 59 due to the reaction force from the shaft body 61 is restricted. As a result, a large moment can be applied to the shaft body 61.

Then, in the manipulator 11, the pair of outer horizontal plate springs is formed integrally with the holder 43. That is, in the holder 43, the holder body 71 and the outer horizontal plate spring are integrally formed without having a joint portion. The holder 43 does not have a joint portion by a fixture, a caulking portion, an adhesive, or the like. In the related art, when the holder body 71 is made of stainless steel, and the outer horizontal plate spring is made of titanium or a titanium alloy, due to the difficulty of welding, the holder body 71 and the outer horizontal plate spring are connected and integrated by the pin 45 or the like (separate members are combined into one). When the holder body 71 is integrated by fixing each of the pair of outer horizontal plate springs with a fixture such as the pin 45, for example, the holder 43 needs at least five components (one holder body 71, two outer horizontal plate springs, and two fixtures). In contrast, the integrally formed holder 43 needs only one component. In the integrally formed holder 43, wear, backlash, or accuracy degradation caused by a joining structure using a fixture or the like does not occur. In addition, body fluids such as blood do not enter the joined structure and can be easily cleaned, and can be used safely in the next and subsequent operations by being sufficiently dried. Furthermore, it is not necessary to downsize individual components when a plurality of separate components are integrated. Therefore, compare to the manipulator 11 having the same size, since the integrally formed holder 43 eliminates a plurality of small components, the risk of strength reduction and the risk of disconnection can be avoided. Long-term use is also possible. As a result, the manipulator 11 in which the holder 43 is integrally formed has a great advantage particularly in the case of a small diameter (outer diameter of approximately 4 mm or less) applied to neurosurgery or like that requires miniaturization.

In addition, in the manipulator 11, the holder body 71 has the cylindrical outer peripheral surface 73, and the pair of outer horizontal plate springs is extended from the cylindrical outer peripheral surface 73 in a direction along the generatrix. The cylindrical outer peripheral surface 73 and the arc-shaped outer peripheral surface 75 of each outer horizontal plate spring have the same radius of curvature. That is, the holder body 71 and the pair of outer horizontal plate springs can be processed from the same metal tube material without providing a joint portion. The holder 43 uses a material (metal tube material) having a circular inner and outer diameter, removes a part thereof and leaves the other part, and accordingly, different rigidity (spring property) can be expressed at a desired site. In the manipulator 11 to be applied to neurosurgery, the outer diameter of the holder 43 inserted inside the outer tube 21 (outer diameter of approximately 4 mm or less) is approximately 3 mm or less. The holder 43 made of titanium or titanium alloy having such a small diameter can be manufactured by cutting a metal tube material by wire cut electric discharge machining, for example.

In addition, in the manipulator 11, each outer horizontal plate spring is formed with a site that is thinner and narrower than both ends in the longitudinal direction (that is, the flexible portion 77) is formed at the central portion in the extending direction. The flexible portion 77 has a smaller cross-sectional area than both ends in the longitudinal direction. Therefore, in the outer horizontal plate spring, the stress acting on both ends in the longitudinal direction concentrates on the flexible portion 77 at the central portion in the extending direction and is bent (elastically deformed). In this manner, by controlling the thickness and the plate width of the outer horizontal plate spring formed integrally with the holder body 71, the holder 43 can be a single component made of the same material, and can express spring properly only at a desired position.

In addition, the pair of outer horizontal plate springs is disposed inside the outer tube 21 such that a part of the extending direction is in contact with the inner wall surface of the outer tube 21. The pair of outer horizontal plate springs is deformed (bent) in a direction inclined toward one outer horizontal plate spring side or in a direction inclined toward the other outer horizontal plate spring. Since the pair of outer horizontal plate springs is in contact with the inner wall surface of the outer tube 21, bending is restricted inside the outer tube 21. The pair of outer horizontal plate springs is bent at a part where most of the pair of outer horizontal plate springs protrudes from the outer tube 21. Therefore, in the pair of outer horizontal plate springs, the open end of the outer tube 21 can be regarded as a bending start end. That is, the opening portion of the outer tube 21 serves as a fulcrum for supporting the outer horizontal plate spring on the bending side. In this manner, in the manipulator 11, a part of the outer horizontal plate spring is made into the inner wall surface of the outer tube 21, and the open end of the outer tube 21 is used as a fulcrum. Accordingly, the outer horizontal plate spring can suppress the useless expansion of a bending radius. The fulcrum moves on the outer horizontal plate spring as the outer horizontal plate spring moves back and forth from the open end of the outer tube 21.

Then, the outer horizontal plate spring and the horizontal plate spring 55 have a smooth surface with no irregularities adjacent to each other in the extending direction at the central portion in the extending direction. Accordingly, the manipulator 11 makes it difficult for body fluid such as blood to adhere to the outer horizontal plate spring or the horizontal plate spring 55. In addition, the attached body fluid such as blood can be easily removed from the outer horizontal plate spring and the horizontal plate spring 55 by cleaning. In addition, in particular, the outer horizontal plate spring can be smoothly moved back and forth from the open end of the outer tube 21.

Furthermore, in the manipulator 11, the deformation of the curved vertical plate spring 59 in the further curved direction is restricted by the guide 87 that comes into contact with the curved outer surface 89. Accordingly, the deformation in the curved direction of the curved vertical plate spring 59 due to the reaction force from the shaft body 61 is restricted. As a result, a large moment can be applied to the shaft body 61.

In addition, in the manipulator 11, the holder 43 is integrally formed of metal or resin. That is, in the holder 43, the holder body 71, and the pair of outer horizontal plate springs are integrally formed of metal or resin. In the present specification, the term "integrally formed" does not include a structure joined by a fixture (bolt, nut, grommet, eyelet, rivet), a caulking portion, adhesives, or the like. In the holder 43, forming integrally includes, for example, a manufacturing method in which unnecessary parts are removed by cutting, grinding, or electrical discharge machining (such as wire cut electric discharge machining), or laser processing of a tube material as a material to obtain a structure having a desired shape (the holder 43). In addition, as an integral formation, for example, the holder 43 having a desired shape may be obtained by filling a mold with a resin material. In this case, two-color molding and insert molding are included. Therefore, "integrally" is synonymous with "integral" in terms of "being in a relationship that cannot be divided into one", but is not required to be made of a single material. Therefore, the integrally formed holder 43 does not have a fixing portion joined using another member.

Next, a modification example of the manipulator 11 of the above-described embodiment will be described.

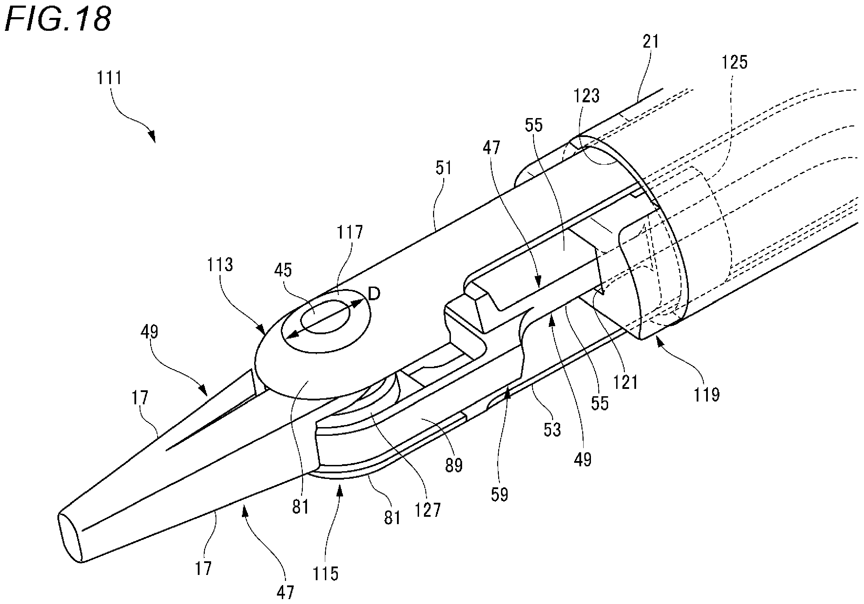

FIG. 18 is an enlarged perspective view of a main part of a tip end portion of a manipulator 111 according to a modification example. In the manipulator 111 of the modification example, the same reference numerals are given to the same members or parts as those in the present embodiment, and duplicate descriptions are omitted.

In the manipulator 111, the above-described holder 43 is divided into a first holder 113 and a second holder 115. The first holder 113 is formed integrally with the first outer horizontal plate spring 51 that supports one end of the pin 45 and is one of the pair of outer horizontal plate springs. The second holder 115 supports the other end of the pin 45 and is integrally formed with the second outer horizontal plate spring 53 that is the other of the pair of outer horizontal plate springs.

The manipulator 111 is separated from the above-described holder 43 into the first holder 113 and the second holder 115 by eliminating the side plate 83 and the guide 87.

In addition, in the manipulator 111, a fixing portion 117 that fixes both ends of the pin 45 to each of the first holder 113 and the second holder 115 is formed in a circular shape having a diameter D (see FIG. 18) larger than approximately half of the width dimension W (see FIG. 3) (for example, W/2 length) orthogonal to the extending direction of the first outer horizontal plate spring 51 and the second outer horizontal plate spring 53.

The fixing portion 117 is preferably larger than the outer diameter of the pin 45. For example, the fixing portion 117 may have a head shape such as a bolt and a rivet formed at one end of the pin 45 or a nut shape that is fixed to the other end of the pin 45 by engagement, bonding, brazing, or welding. The holding plate 81 of each of the first holder 113 and the second holder is preferably formed with a concave portion having the fixing portion 117 fitted inward and having the same surface as the holding plate 81.

In addition, the manipulator 111 is provided with a spring distribution portion 119 at the tip end of the outer tube 21. A guide hole 121 is formed through the spring distribution portion 119 so as to be coaxial with the outer tube 21. The horizontal plate spring 55 of each of the first flexible operating body 47 and the second flexible operating body 49 is inserted into the guide hole 121. The guide hole 121 suppresses the eccentricity of the inserted horizontal plate spring 55 in the outer tube 21 that is displaced in the radial direction.

In addition, by providing the spring distribution portion 119 at the tip end, the outer tube 21 has a pair of spring position restricting gaps 123 formed at an opening at the tip end with the spring distribution portion 119 interposed therebetween. The first outer horizontal plate spring 51 and the second outer horizontal plate spring 53 are inserted through each of the pair of spring position restricting gaps 123. The spring position restricting gap 123 restricts the displacement of the first outer horizontal plate spring 51 and the second outer horizontal plate spring 53 toward the radially inner side of the outer tube 21.

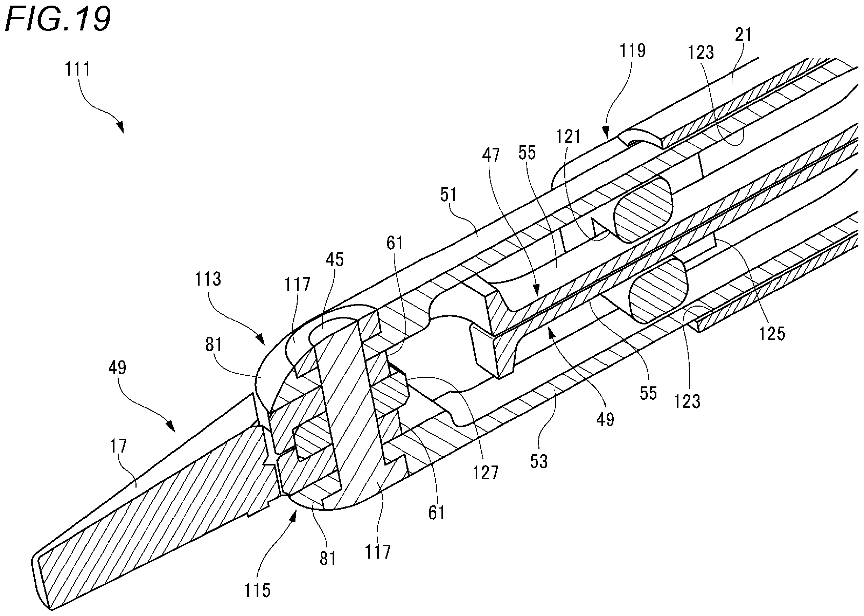

FIG. 19 is a side sectional view of the manipulator shown in FIG. 18. The spring distribution portion 119 can be formed integrally with the outer tube 21. In addition, the spring distribution portion 119 may be fixed by engaging, bonding, brazing, or welding a member formed separately from the outer tube 21. When the spring distribution portion 119 is provided separately from the outer tube 21, a fixing margin can be secured by forming a flange portion 125 for fixing to the inner wall surface of the outer tube 21.

In the first flexible operating body 47 and the second flexible operating body 49, a spacer 127 may be disposed between the shaft bodies 61 through which the pin 45 passes. The spacer 127 can reduce friction caused by relative rotation between the shaft bodies 61 when the first flexible operating body 47 and the second flexible operating body 49 open and close, and can smooth the opening and closing operation.

Next, the operation of the manipulator 111 of the modification example will be described.

In the manipulator 111 of the modification example, the holder 43 is divided into the first holder 113 and the second holder 115. The divided first holder 113 and second holder 115 interpose the horizontal plate springs 55 of the flexible operating body in parallel between the first holder 113 and the second holder 115. Between the first holder 113 and the second holder 115, the respective curved vertical plate springs 59 connected to the pair of horizontal plate springs 55 are disposed. The curved vertical plate springs 59 are pushed and pulled in different directions by the pair of horizontal plate springs 55, respectively. In the manipulator 111, as shown in FIG. 12, the operation portion 17 is displaced in the Y direction and the -Y direction by pushing and pulling the pair of horizontal plate springs 55 in different directions. At this time, one of the curved vertical plate springs 59 on the side opposite to the tilting direction may be deformed outward and bulge out. In the curved vertical plate spring 59 bulging outwards, the curved outer surface 89 may come into contact with the guide 87 (see FIG. 2) of the holder 43. Friction occurs as the curved outer surface 89 is rubbed with the guide 87. If the friction is excessive, there is a possibility that the tilting accuracy of the operation portion 17 may be lowered, or the curved vertical plate spring 59 may be destroyed.

In contrast, in the manipulator 111 in which the holder 43 is divided into a first holder and a second holder, the guide 87 that integrally links both is eliminated. Therefore, in the divided first holder 113 and second holder 115, friction caused by the curved vertical plate spring 59 and the guide 87 rubbing each other does not occur. Accordingly, with the manipulator 111, the tilting accuracy of the operation portion 17 can be improved, and the destruction of the curved vertical plate spring 59 due to friction can be prevented. In addition, with the manipulator 111, the manufacture becomes easier as compared with the structure having the integral holder 43 linked by the guide 87. Furthermore, compared to the integral holder 43, the friction with the guide 87 is eliminated, so that the tilting of the operation portion 17 can be made smooth.

In addition, in the manipulator 111, the pin 45 passes through the tip ends (the holding plate 81) of the divided first holder 113 and second holder 115. Both ends of the pin 45 are fixed to the holding plate 81 by the fixing portions 117, respectively. The fixing portion 117 if formed in a circular shape having a diameter D larger than approximately half of the width dimension W (for example, half W/2) orthogonal to the extending direction of the outer horizontal plate spring. Therefore, the first holder 113 and the second holder 115 can fix the pin 45 from the holding plate 81 with high strength compared to the structure in which both ends of the pin 45 are fixed by a fixing portion having a diameter smaller than the half W/2 of the width dimension W of the outer horizontal plate spring. Accordingly, the fixing strength of the pin 45 can be enhanced in response to the stress acting on the fixing structure of both ends of the pin 45 and the holding plate 81 which is increased by eliminating the guide 87.

In addition, in the manipulator 111, since the spring distribution portion 119 is provided at the tip end of the outer tube 21, the horizontal plate spring 55 of each of the first flexible operating body 47 and the second flexible operating body 49 is inserted into the guide hole 121. At the same time, the first outer horizontal plate spring 51 and the second outer horizontal plate spring 53 can be guided through the pair of spring position restricting gaps 123, respectively. Accordingly, the displacement in the radial direction of the outer tube 21 in the pair of horizontal plate springs 55, the first outer horizontal plate spring 51, and the second outer horizontal plate spring 53 can be suppressed. As a result, the operation accuracy of opening and closing and tilting of the operation portion 17 can be enhanced.

Therefore, the manipulator 11 according to the present embodiment can eliminate a wire, can be reduced in size, weight, and cost with a small number of components, and can be easily approached to the target lesion.

Although the embodiments have been described above with reference to the drawings, it is needless to say that the present disclosure is not limited to such examples. It will be apparent to those skilled in the art that various changes, modifications, and combinations can be conceived within the scope of the claims, and it is understood that they naturally belong to the technical scope of the present disclosure.

In the embodiment described above, the manipulator may have three or more flexible operating bodies.

This application is based on Japanese patent application No. 2017-092574, filed on May 8, 2017, the contents of which are incorporated herein by reference.

### INDUSTRIAL APPLICABILITY

The present disclosure is useful as a manipulator that can eliminate a wire as a tool for transmitting power to a tip end portion that performs treatment on a target lesion, can be reduced in size, weight, and cost with a small number of components, and can be easily approached to the target lesion.

### REFERENCE SIGNS LIST

- 11:: manipulator
- 17:: operation portion
- 21:: outer tube
- 43:: holder
- 45:: pin
- 47:: first flexible operating body (flexible operating body)
- 49:: second flexible operating body (flexible operating body)
- 51:: first outer horizontal plate spring (outer horizontal plate spring)
- 53:: second outer horizontal plate spring (outer horizontal plate spring)
- 55:: horizontal plate spring
- 59:: curved vertical plate spring
- 61:: shaft body
- 65:: R portion (bent portion)
- 67:: rotation center
- 73:: cylindrical outer peripheral surface
- 75:: arc-shaped outer peripheral surface
- 77:: flexible portion
- 87:: guide
- 89:: curved outer surface
- 111:: manipulator
- 113:: first holder
- 115:: second holder
- 117:: fixing portion

## Claims

1. A manipulator comprising:
a pair of flexible operating bodies including
a rectangular horizontal plate spring,
a curved vertical plate spring which stands upright with a plate surface perpendicular to a plate surface of the horizontal plate spring, wherein a proximal end of the curved vertical plate spring is connected to one end of the horizontal plate spring in a longitudinal direction, the curved vertical plate spring protrudes to one side in a plate width direction of the horizontal plate spring, and extends along the longitudinal direction of the horizontal plate spring, and wherein a tip end of the curved vertical plate spring in an extending direction is a bent portion bent in a direction opposite to a direction of the protruding,
a shaft body which is connected to a tip end of the bent portion and is supported to be rotatable at a rotation center perpendicular to the plate surface of the horizontal plate spring, and
an operation portion which is provided to protrude in a radial direction from an outer periphery of the shaft body; and
a holder which supports both ends of a pin passing through the shaft body of each of the pair of flexible operating bodies and links the pair of flexible operating bodies to be rotatable about the rotation center, wherein
the holder accommodates the horizontal plate springs of the pair of flexible operating bodies to be interposed in parallel between the holder, and
a pair of outer horizontal plate springs extending in the same direction as the horizontal plate spring is integrally formed with the holder.

2. The manipulator according to claim 1, wherein the pair of outer horizontal plate springs extends from a cylindrical outer peripheral surface of the holder in a direction along a generatrix and has an arc-shaped outer peripheral surface having the same radius of curvature as the cylindrical outer peripheral surface.

3. The manipulator according to claim 1 or 2, wherein a flexible portion that is thinner and narrower than both ends of the outer horizontal plate spring in the longitudinal direction is formed at a central portion of the outer horizontal plate spring in an extending direction.

4. The manipulator according to any one of claims 1 to 3, wherein the pair of outer horizontal plate springs is disposed inside an outer tube such that a part in an extending direction is in contact with the inner wall surface of the outer tube.

5. The manipulator according to any one of claims 1 to 4, wherein the outer horizontal plate spring and the horizontal plate spring have a smooth surface with no irregularities adjacent to each other in an extending direction at a central portion in the extending direction.

6. The manipulator according to any one of claims 1 to 5, wherein the holder includes a pair of guides that comes into contact with a curved outer surface of the curved vertical plate spring of each of the pair of flexible operating bodies.

7. The manipulator according to any one of claims 1 to 6, wherein the holder and the pair of outer horizontal plate springs are integrally formed of either one of metal or resin.

8. The manipulator according to claim 1, wherein the holder is divided into a first holder that supports one end of the pin and is integrally formed with one of the pair of outer horizontal plate springs, and a second holder that supports the other end of the pin and is integrally formed with the other of the pair of outer horizontal plate springs.

9. The manipulator according to claim 8, wherein a fixing portion that fixes both ends of the pin to each of the first holder and the second holder is formed in a circular shape having a diameter larger than approximately half of a width dimension orthogonal to an extending direction of the outer horizontal plate spring.
